# EUROPEAN PATENT APPLICATION

(11) **EP 2 269 688 A1**
(43) Date of publication of application: **05.01.2011**
(21) Application number: 10164938.2
(22) Date of filing: 04.06.2010
(51) Int. Cl.: A61N 1/08, G06K 19/073

(54) **Transient voltage suppression circuit for an implanted RFID chip**

(30) Priority: 01.03.2010 US 715151; 24.09.2009 US 566223; 24.09.2009 US 566490; 02.07.2009 US 497424
(71) Applicant: Greatbatch Ltd., Clarence, New York 14031 (US)
(72) Inventor: Stevenson, Robert A., Canyon Country, CA 91387 (US); Frysz, Christine A., Orchard Park, NY 14127 (US)
(74) Representative: Richter, Werdermann, Gerbaulet & Hofmann

(57) **Abstract**

A transient voltage suppressing (TVS) circuit includes an implantable RFID chip, an antenna associated with the RFID chip, and a transient voltage suppressor electrically connected in parallel to both the RFID chip and the antenna. The transient voltage suppressor may be formed of an array of diodes, such as back-to-back diodes, at least one Zener diode, or back-to-back or series opposing Zener diodes. In preferred embodiments, the antenna is formed of a biocompatible material suitable for long-term exposure to body tissue and body fluids, and the RFID chip and the transient voltage suppressor are disposed within a hermetically sealed biocompatible container.

## Description

### Background of the Invention

This invention relates generally to high voltage circuit protection of implantable and biocompatible radio frequency identification (RFID) tags and associated antennas which may be used with medical devices or for general personal identification purposes. More particularly, high voltage or transient voltage suppression (TVS) circuits are described which protect the sensitive RFID microchip from shorting out in the presence of an over-voltage such as caused by some types of surgical equipment and automatic external defibrillators (AEDs).

There are known in the art various methods for identifying implanted medical devices. One such method is the use of X-ray identification tags encapsulated within header blocks of pacemakers or implantable cardioverter defibrillators (ICD). Such X-ray identification tags can be read on an X-ray of the implanted device and provide information to the physician. The information so provided is limited due to space and typically includes only the manufacturer and model number of the implanted device.

It would be beneficial if physicians were able to obtain additional information about an implanted device and/or a patient from an implanted identification tag. Such beneficial information includes, in addition to the manufacturer and model number of the device, the serial number of the device, the treating physician's name and contact information and, if authorized by the patient, the patient's name, contact information, medical condition and treatment, and other relevant information.

Currently, most active implantable medical device (AIMD) patients carry some sort of identification. This could be in the form of a card carried in the wallet or an ID bracelet indicating, for example, that they are a pacemaker wearer of a certain model and serial number. However, such forms of identification are often not reliable. It is quite common for an elderly patient to be presented at the emergency room (ER) of a hospital without their wallet and without wearing any type of a bracelet. In addition, there have been a number of situations where the patient (due to dementia or Alzheimer's, etc.) cannot clearly state that he or she even has a pacemaker.

Oftentimes the ER physician will palpitate the patient's chest and feel that there is an implanted device present. If the patient is comatose, has low blood pressure, or is in another form of cardiac distress, this presents a serious dilemma for the ER. At this moment in time, all that the ER knows is that the patient has some sort of an AIMD implant in his or her chest. It could be a pacemaker, a cardioverter defibrillator, or even a vagus nerve stimulator or deep brain stimulator.

What happens next is both laborious and time consuming. The ER physician will have various manufacturers' internal programmers transported from the hospital cardiology laboratory down to the ER. ER personnel will then try to interrogate the implantable medical device to see if they can determine what it is. For example, they might first try to use a Medtronic programmer to see if it is a Medtronic pacemaker. Then they might try a St. Jude, a Guidant, an ELA, a Biotronik or one of a number of other programmers that are present. If none of those programmers work, then the ER physician has to consider that it may be a neurostimulator and perhaps go get a Cyberonics or Neuropace programmer.

It would be a great advantage and potentially lifesaving if the ER physician could very quickly identify the type of implant and model number. In certain cases, for example, with a pacemaker patient who is in cardiac distress, with an external programmer they could boost the pacemaker output voltage to properly recapture the heart, obtain a regular sinus rhythm and stabilize blood pressure. All of the lost time running around to find the right programmer, however, generally precludes this. Accordingly, there is a need for a way to rapidly identify the type and model number of an active implantable medical device so that the proper external programmer for it can be rapidly identified and obtained.

It is also important to note that lead wire systems generally remain in the human body much longer than the active implantable medical device itself. For example, in the case of a cardiac pacemaker, the cardiac pacemaker batteries tend to last for 5 to 7 years. It is a very difficult surgical procedure to actually remove leads from the heart once they are implanted. This is because the distal TIP and other areas of the leads tend to become embedded and overgrown by tissue. It often takes very complex surgical procedures, including lasers or even open heart surgery, to remove such lead wire systems. When a pacemaker is replaced, the pectoral pocket is simply reopened and a new pacemaker is plugged into the existing leads. However, it is also quite common for leads to fail for various reasons. They could fail due to breakdown of electrical insulation or they could migrate to an improper position within the heart. In this case, the physician normally snips the leads off and abandons them and then installs new leads in parallel with the old abandoned leads.

Abandoned leads can be quite a problem during certain medical diagnostic procedures, such as MRI. It has been demonstrated in the literature that such leads can greatly overheat due to the powerful magnetic fields induced during MRI. Accordingly, it is important that there be a way of identifying abandoned leads and the lead type. Also, there is a need to identify such abandoned leads to an Emergency Room physician or other medical practitioner who may contemplate performing a medical diagnostic procedure on the patient such as MRI. This is in addition to the need to also identify the make and model number of the active implantable medical device.

It is also important to note that certain lead systems are evolving to be compatible with a specific type of medical diagnostic procedure. For example, MRI systems vary in static field strength from 0.5 Tesla all the way above 10 Tesla. A very popular MRI system, for example, operates at 3 Tesla and has a pulse RF frequency of 128 MHz. There are specific certain lead systems that are evolving in the marketplace that would be compatible with only this type of MRI system. In other words, it would be dangerous for a patient with a lead wire designed for 3 Tesla to be exposed to a 1.5 Tesla system. Thus, there is also a need to identify such lead systems to Emergency Room and other medical personnel when necessary. For example, a patient that has a lead system that has been specifically designed for use with a 3 Telsa MRI system may have several pacemaker replacements over the years.

It is already well known in the prior art that RFID tag implants can be used for animals, for example, for pet tracking. It is also used in the livestock industry. For example, RFID tags can be placed in cattle to identify them and track certain information. An injectable RFID tag for humans has also been developed. However, none of the current RFID tags have been designed to have long term reliability, hermeticity, and biocompatibility within the body fluid environment.

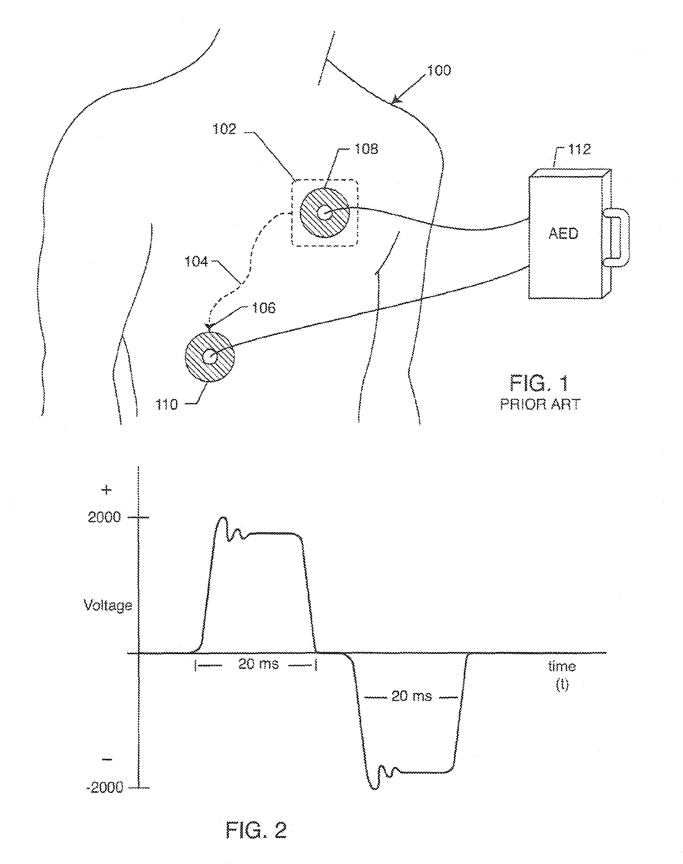

FIG. 1 is an outline drawing of the neck and torso of a typical patient 100 who has an active implanted medical device (AIMD 102). In this case, by way of illustration, the AIMD is a pacemaker. The pacemaker 102 has an implanted lead 104 which is directed to a distal electrode 106 which, in this case, would be typically implanted into the right ventricle of the patient's heart. The pacemaker 102 typically does sensing and also provides pacing pulses in order that the heart can properly beat. In case of a cardiac emergency, for example when the patient would stop breathing or stop having a heart beat, emergency personnel could place the two electrode paddles 108 and 110 of an automatic external defibrillator (AED) 112 as shown. When one carefully reads the instructions on the lid of an AED 112, it shows a diagram for correct placement of the paddles. Typically, one paddle would be placed down fairly low in the abdomen and the other paddle would be placed fairly high on the chest. However, in haste, emergency personnel often place one paddle directly over the pectoral pocket area of the cardiac pacemaker 102 and the other paddle directly over the right ventricle of the heart. When the paddles are placed in these (incorrect) locations, maximum currents are induced into the implanted lead 104. These induced currents are undesirable as they could cause excessive currents to flow inside the pacemaker 102 thereby damaging lead-based sensitive electronic circuits. To protect against such surge currents from an AED 112, most AIMDs have internal circuit protection devices.

However, it is now becoming quite common for electronic circuits to be placed in the lead 104 itself. Absent the present invention, there is no protection for these electronic components against the high voltage current surges caused from AEDs or AED events.

FIG. 2 shows a typical biphasic shock waveform where the AED voltage will vary from +2000 to -2000 volts. The timing of the pulses can vary greatly from one AED manufacturer to another. In one typical example, the positive going pulse would have a pulse width of 20 milliseconds. After a short dwell period, the negative pulse would also have a duration of approximately 20 milliseconds. The biphasic shock waveform of FIG. 2 could also represent the output pulse from an ICD. However, for an ICD, the voltage is typically lower (typically around 800 volts) because the implanted leads are directly connected to heart tissue. The AED has to provide higher energy since it is shocking through the chest wall, pectoral muscles and so forth. Therefore, an ICD is more efficient with its direct connection. However, in both cases, the transient voltage can result in very high surge currents which can be very damaging to active or passive lead-based electronic circuits.

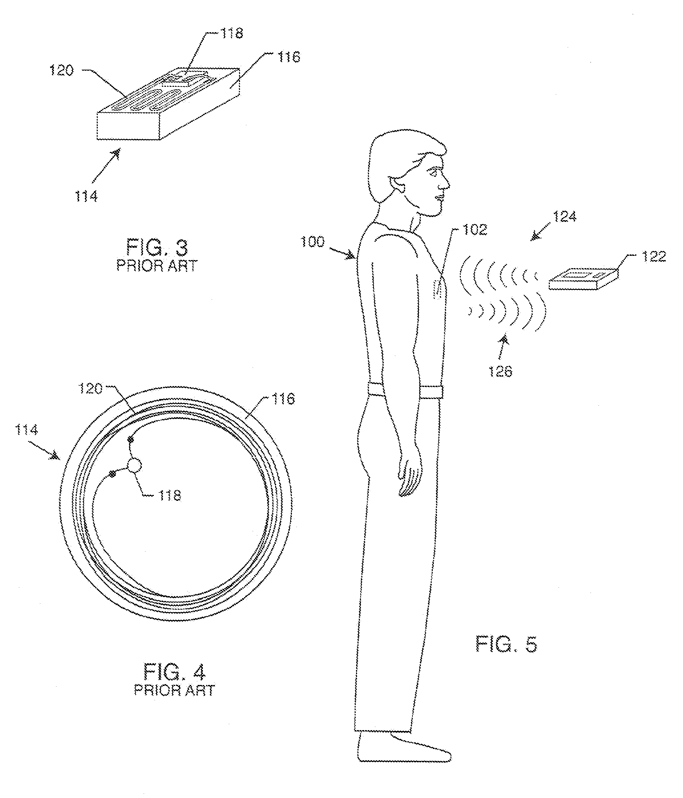

With reference to FIGS. 3 and 4, RFID tags 114 typically involve a small rigid or flexible substrate 116 on which a microelectronic chip 118 is placed along with an embedded or printed antenna 120. These antennas can be Wheeler spirals, rectangles, dipoles, folded dipoles, solenoids or other shapes. The read range of such antennas, particularly for low frequency (LF) and high frequency (HF) readers tends to be very short. That is, the RFID reader has to be in very close proximity to the RFID chip. In order to extend the read range, a larger loop style antenna 120 involving multiple turns, as illustrated in FIG. 4, is typically used. These involve very fine wire, multiple turns of wire, which are then connected to the RFID chip 118.

An implanted RFID chip 118 is always associated with some sort of an implanted antenna 120. In general, the low voltage RFID microchip is very sensitive and can be easily damaged by over-voltages. This is normally not a problem in a general RFID chip environment where it might be used for inventory control, article tracking, or the like. However, implanted antennas and leads within a human body are often subjected to high voltage insults. An increasingly common high voltage insult results from the use from an automatic external defibrillator 112 as described in connection with FIG. 1. However, this is not the only type of high voltage insult that to which an implanted RFID tag 114 with its associated antenna 120 and microchip 118 may be exposed. Other types of high voltage insults can come from various types of hospital diagnostic procedures, such as diathermy, lipotripsy and the like. Another very common type of high voltage insult occurs during surgical procedures that use electro-cautery knives, such as the Bovi knifes. These types of RF cutting scalpels can generate a high voltage particularly if the scalpel is inadvertently touched-off near or adjacent to the AIMD 102 or the implanted RFID tag 114. RFID chips 118 can also be damaged during original installation and handling through static electricity. Static electricity discharges can be of several thousand or even tens of thousands of volts and tend to be very fast acting and short in duration.

Accordingly, there is a need for some type of means for protecting the sensitive RFID microchip 118 from shorting out in the presence of an over-voltage such as caused by, for example, some types of surgical equipment and automatic external defibrillators (AEDs). Such protective means must not interfere with active implanted medical devices or associated circuitry or leads. Moreover, the means employed to solve the problem must be suitable for long-term exposure to body tissue or body fluids. The present invention fulfills these needs and provides other related advantages.

### SUMMARY OF THE INVENTION

In general, the present invention is directed to a system for identifying implants within a patient, comprising an implantable medical device, a radio frequency identification (RFID) tag having a hermetically sealed chip and biocompatible antenna and being associated with the implantable medical device, the RFID tag containing information relating to the patient and/or the implantable medical device, and an interrogator capable of communicating with the RFID tag. More particularly, the present invention is directed to transient voltage suppression circuits which protect the sensitive RFID microchip from damage or shorting out in the presence of an over-voltage such as that which could be caused by hospital, diagnostic or surgical equipment or by an automatic external defibrillator (AED). More specifically, a transient voltage suppression (TVS) circuit is provided for an implanted RFID chip. The TVS circuit comprises an implantable RFID chip, an antenna associated with the RFID chip, and a transient voltage suppressor electrically connected in parallel to both the RFID chip and the antenna.

The transient voltage suppressor preferably comprises an array of diodes which may include back-to-back diodes or back-to-back or series opposing Zener diodes.

The antenna preferably comprises a biocompatible material suitable for long-term exposure to body tissue or body fluids.

A hermetically sealed biocompatible container is provided which is suitable for long-term exposure to body tissue or body fluids, in which the RFID chip and the transient voltage suppressor are disposed. In some preferred embodiments, the hermetically sealed biocompatible container is disposed within a header or an active implantable medical device (AIMD). The antenna may be disposed about the hermetically sealed biocompatible container which itself may be designed such that the RFID chip and the transient voltage suppressor are mechanically disposed in line within the hermetically sealed biocompatible container and yet electrically connected in parallel.

The RFID chip may further be associated with an implantable sensor or stimulator such as a deep brain sensor or stimulator.

Typical AIMDs with which the transient voltage suppression circuit for an implanted RFID chip is associated include medical devices such as a cardiac pacemaker, an implantable defibrillator, a congestive heart failure device, a hearing implant, a cochlear implant, a neurostimulator, a drug pump, a ventricular assist device, an insulin pump, a spinal cord stimulator, an implantable sensing system, a deep brain stimulator, an artificial heart, an incontinence device, a vagus nerve stimulator, a bone growth stimulator, a gastric pacemaker, a Bion, or a prosthetic device and component parts thereof, including lead wires or abandoned lead wires. The active implantable medical device may include a non-metallic header or connector block in which the RFID tag is implanted. The RFID tag may be disposed within the non-hermetically sealed portion, such as the header block, of the medical device. In one embodiment, the RFID chip includes information pertaining to the medical device.

Other features and advantages of the present invention will become apparent from the following more detailed description, taken in conjunction with the accompanying drawings which illustrate, by way of example, the principles of the invention.

### BRIEF DESCRIPTION OF THE DRAWINGS

The accompanying drawings illustrate the invention. In such drawings:
FIG. 1 is an outline drawing of the neck and torso of a typical patient wherein the electrode paddles of an automatic external defibrillator (AED) have been applied over the chest and abdomen.
FIG. 2 is a graph illustrating a typical biphasic shock waveform generated by the AED of FIG. 1.
FIG. 3 is a perspective view of one type of typical prior art RFID tag.
FIG. 4 illustrates another type of typical prior art RFID tag having a large loop-style antenna.
FIG. 5 is an outline drawing of an adult male pacemaker patient and an RFID interrogator.
FIG. 6 is a wire form diagram of a generic human body showing a number of implanted medical devices.
FIG. 7 illustrates a patient in a hospital room and a medical practitioner rushing to the patient with an automatic external defibrillator (AED).
FIG. 8 is an electrical schematic diagram illustrating a transient voltage suppression (TVS) circuit embodying the present invention, which includes an array of two diodes.
FIG. 9 is an electrical schematic diagram similar to FIG. 8, except that the antenna is not shown and that the back-to-back diodes are shown is series.
FIG. 10 is an electrical schematic diagram similar to FIG. 9, except that the back-to-back diodes are shown as a single chip.
FIG. 11 is an electrical schematic diagram similar to FIGS. 8-10, except that the back-to-back diodes have been replaced with a back-to-back Zener diode.
FIG. 12 is a perspective illustration of a typical AIMD, such as a cardiac pacemaker, including a non-hermetically sealed RFID tag encapsulated within the molded header block.
FIG. 13 is a perspective view similar to FIG. 12, except that the RFID chip is disposed within a hermetically sealed biocompatible container and is associated with a biocompatible multi-turn loop antenna.
FIG. 14 is an enlarged view of the RFID tag of FIG. 13, taken generally along the line of the area 14-14 from FIG. 13.
FIG. 15 is an enlarged, exploded perspective view of the RFID chip assembly and hermetic container of FIGS. 13 and 14.
FIG. 16 is a perspective illustration similar to FIG. 13, illustrating a solenoid-type RFID tag embedded within the header block.
FIG. 17 is an enlarged view of the solenoid-type RFID tag taken generally of the area indicated by line 17-17 from FIG. 16.
FIG. 18 is an enlarged sectional view taken generally along the line 18-18 from FIG. 17.
FIG. 19 is an enlarged sectional view taken generally along the line 19-19 from FIG. 17, illustrating arrangement of components within elongated biocompatible and hermetically sealed housing.
FIG. 20 is a schematic illustration of the circuit connections for the electronic components of FIG. 19.
FIG. 21 is an electrical schematic diagram of the components of FIGS. 19 and 20.
FIG. 22 is an electrical schematic diagram equivalent for that shown in FIG. 21.
FIG. 23 illustrates the relative size of the hermetically sealed container for the RFID tag and associated TVS circuit of FIGS. 17 and 19 in comparison with a United States penny.
FIG. 24 is a cross-sectional view of a human head and skull showing an implanted deep brain stimulation electrode assembly.
FIG. 25 is an enlarged sectional view taken generally of the area indicated by the line 25-25 from FIG. 24.
FIG. 26 is an enlarged electrical schematic illustration of the components associated with the deep brain electrode assembly taken generally of the area indicated by line 26-26 from FIG. 25.

### DETAILED DESCRIPTION OF THE PREFERRED EMBODIMENTS

The present invention is directed to a radio frequency identification (RFID) system for use with active implantable medical devices (AIMDs) 102 and an associated RFID tag 114. Specifically, the RFID system comprises an RFID tag 114 implanted in a patient's body and associated with an implanted AIMD 102 or component, and an interrogator 122 in communication with the RFID tag 114.

More particularly, the present invention resides in circuit protection devices for RFID microchips 118. Such circuit protection devices can be a diode, a Zener diode, an avalanche diode, Zener connected series opposing (back-to-back) diodes, or just a general TVS diode. Transient voltage suppression diodes are electronic components used to protect sensitive circuits from voltage spikes induced on connected wires. In the case of an RFID chip 118, the connected wire is its own antenna. TVS diodes are also commonly referred to as transorbs after the brand name TransZorb, registered by General Semiconductor (now part of Vishay). These devices operate by shunting excess current when the induced voltage exceeds the avalanche breakdown potential. TVS devices act as clamping devices, suppressing all over-voltages above its breakdown voltage. Like all clamping devices, the TVS automatically resets when the over-voltage goes away, but absorbs much more of the transient energy internally than a similarly rated crowbar device.

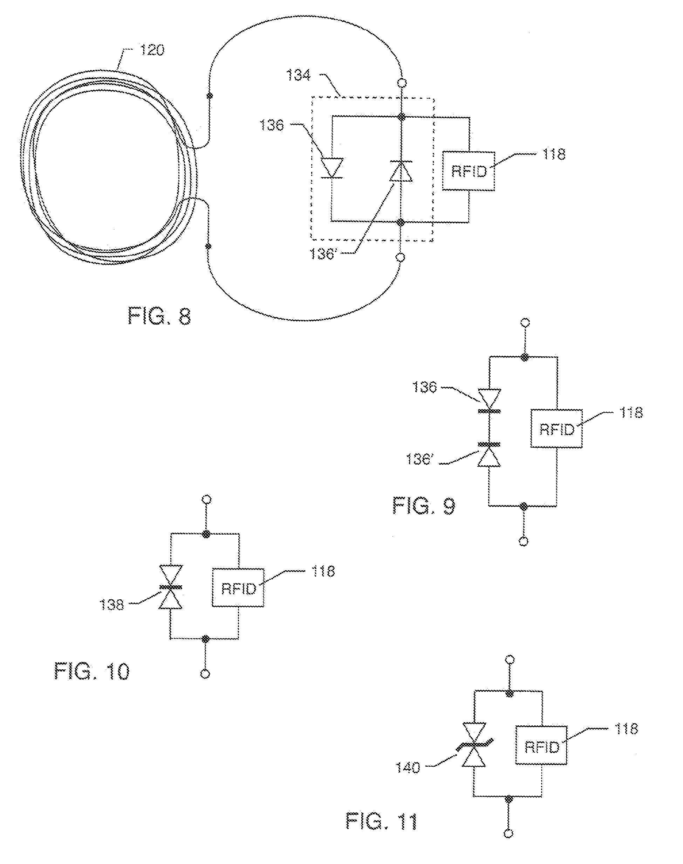

TVS suppression diodes may be either unidirectional or bidirectional. The unidirectional device acts as a rectifier in the forward direction, like any other avalanche diode, but is made and tested to handle very large peak currents. In a preferred embodiment, a bidirectional TVS suppression diode is represented by two mutual opposing avalanche diodes in series with one another and connected in parallel with the circuit to be protected (FIG. 9). While this representation is schematically accurate, physically the devices are now manufactured as a single component (FIG. 10). A TVS diode can respond to over-voltages faster than other common over-voltage protection components, such as fuses, varistors or gas discharge tubes. The actual clamping occurs in roughly 1 picosecond (ignoring circuit inductance). This makes TVS suppression diodes useful protection against very fast and often damaging voltage transients.

Fast transients are typically associated with the high voltage output of an implantable cardioverter defibrillator, an AED, or certain surgical knives such as the Bovi knife. The TVS diodes are also fast enough to protect against electrostatic discharge. As used herein, the term TVS or TVS diode shall be inclusive of all types of circuit protection diodes, Zener diodes, avalanche diodes, back-to-back diodes or avalanche or Zener diodes connected series opposing.

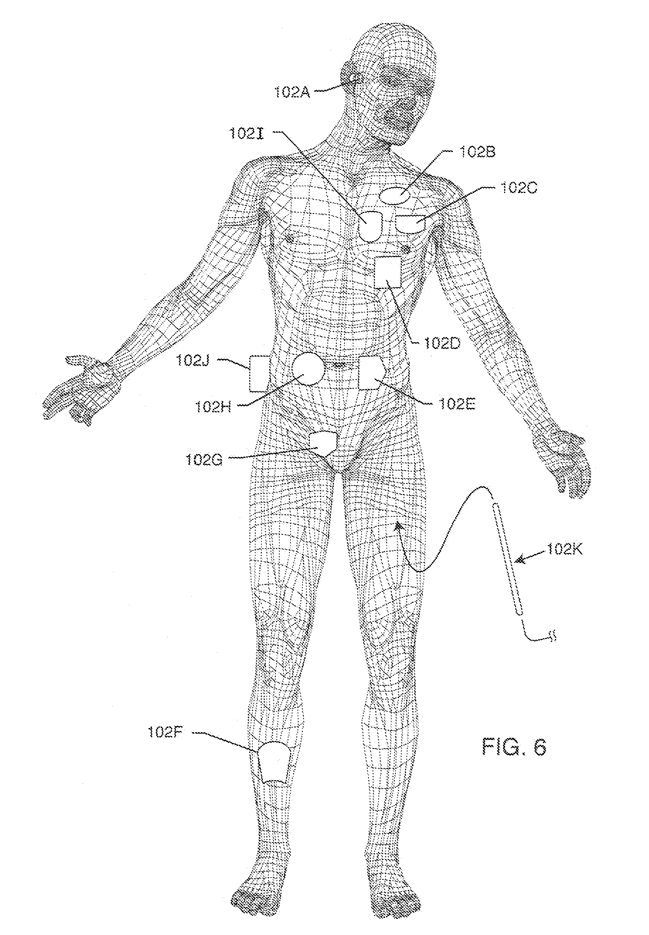

FIG. 5 is an outline drawing of an adult male pacemaker patient with an AIMD. A potential location for an AIMD 102 is shown by a dashed ellipse 102, which is typical of a right or left pectoral muscle implant. Right and left pectoral muscle implants are typical for a cardiac pacemaker or implantable cardioverter defibrillator (ICD). The right and left pectoral muscle region is chosen due to the easy access to the cephalic or subclavian veins for insertion of lead wires and electrodes down into the heart. The present invention has application in a wide variety of AIMDs such as those shown in FIG. 6.

Referring once again to FIG. 5, one can see an RFID interrogator 122, also known as a hand held scanner or reader. The interrogator 122 transmits an electromagnetic field pulse 124 which is intercepted by the antenna 120 that is part of the implanted RFID tag 114. The implanted RFID tag 114 is generally passive. That means that it does not have its own self-contained source of energy such as a battery (although it can). The electromagnetic field pulse 124 that comes from the interrogator 122 resonates with the antenna 120 and RFID chip 118 providing energy for the RFID chip to generate a signal and the antenna 120 to emit a return pulse 126. This pulse 126 is picked up by an antenna in the interrogator 122. The pulse 126 contains digital modulation which can include information such as the model number of the patient's AIMD, the serial number of the AIMD, the manufacturer of the lead wire system, the name of the patient's physician, and contact information for the physician. In addition, if the patient authorizes, the digital pulse can also contain the patient's name, the patient's medical condition, the patient's address and telephone number, and other pertinent information.

FIG. 6 is a wire formed diagram of a generic human body showing a number of implanted medical devices 102A-K. 102A represents a family of hearing devices which can include the group of cochlear implants, piezoelectric sound bridge transducers and the like. 102B represents a variety of neurostimulators and brain stimulators. Neurostimulators are used to stimulate the Vagus nerve, for example, to treat epilepsy, obesity and depression. Brain stimulators are pacemaker-like devices and include electrodes implanted deep into the brain for sensing the onset of the seizure and also providing electrical stimulation to brain tissue to prevent the seizure from actually occurring. The leadwires associated with a deep brain stimulator are often placed using real time MRI imaging. Most commonly such leadwires are placed during real time MRI. 102C shows a cardiac pacemaker which is well-known in the art. 102D includes the family of left ventricular assist devices (LVAD's), and artificial hearts, including the recently introduced artificial heart known as the Abiocor. 102E includes an entire family of drug pumps which can be used for dispensing of insulin, chemotherapy drugs, pain medications and the like. Insulin pumps are evolving from passive devices to ones that have sensors and closed loop systems. That is, real time monitoring of blood sugar levels will occur. These devices tend to be more sensitive to EMI than passive pumps that have no sense circuitry or externally implanted leadwires. 102F includes a variety of bone growth stimulators for rapid healing of fractures. 102G includes urinary incontinence devices. 102H includes the family of pain relief spinal cord stimulators and anti-tremor stimulators. 102H also includes an entire family of other types of neurostimulators used to block pain. 102I includes a family of implantable cardioverter defibrillators (ICD) devices and also includes the family of congestive heart failure devices (CHF). This is also known in the art as cardiac resynchronization therapy devices, otherwise known as CRT devices. 102J illustrates an externally worn pack. This pack could be an external insulin pump, an external drug pump, an external neurostimulator or even a ventricular assist device. 102K illustrates the insertion of an external probe or catheter. These probes can be inserted into the femoral artery, for example, or in any other number of locations in the human body.

RFID standards are evolving worldwide at various frequencies generally between 125 kHz and 915 MHz. For example, a 915 MHz protocol is generally evolving to be used for retail goods and inventory control. However, due to the high frequency, the 915 MHz protocols are not very useful for human implants. The reason for this is that humans are largely water and 915 MHz fields are greatly affected by the presence of water. The preferred embodiment is another RFID protocol which operates at 13.56 MHz which is ideal for an implantable RFID tag 114. The 13.56 MHz lower frequency will readily penetrate and communicate with the tag instead of reflecting off of the skin surface or being absorbed. There are other lower frequency RFID systems, for example, in the 125 to 135 kHz range which would also be ideal.

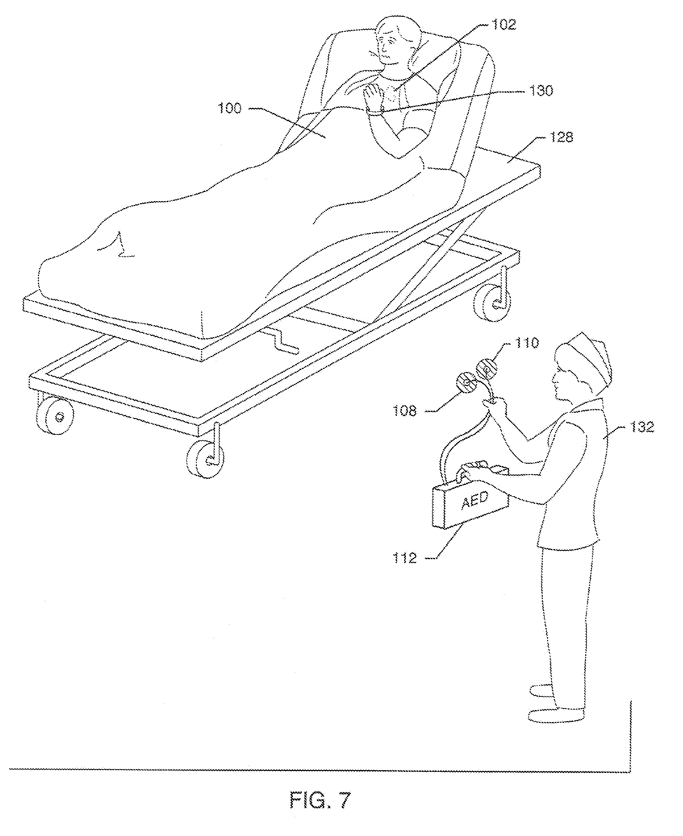

FIG. 7 illustrates a hospitalized patient 100 who is lying in a gurney or hospital bed 128. This particular patient may have an RFID enabled wristband 130. The patient may also have an implanted AIMD 102, such as a cardiac pacemaker. There could be an RFID tag 114 associated with AIMD 102 as well. This particular patient 100 is in severe cardiac distress and has a dangerous ventricular arrhythmia. This has set off monitors in the hospital room. A medical practitioner 132 is rushing to the patient 100 with an automatic external defibrillator (AED) 112. Associated with the AED are shocking electrodes 108 and 110 which will be placed on the patient's chest. Then a high voltage shock will be delivered hopefully to restore sinus rhythm to the patient's heart. The high voltage associated with the AED 112 could damage the RFID chip 118 associated with patient wristband 130 and/or associated with the AIMD 102. In accordance with the present invention, TVS circuits are used to protect the RFID chips so that it will not burn out during the high voltage AED event.

FIG. 8 is a schematic diagram showing the RFID microchip 118 and the associated RFID antenna120. The antenna 120 is connected to the two terminals of the RFID microchip 118. Not shown is a capacitor which is generally in parallel with the RFID chip which gathers energy from an external reader. In accordance with the present invention, a TVS device 134 is wired in parallel with RFID chip 118 and the antenna120. In FIG. 8, the TVS device 134 is shown as parallel wired back-to-back avalanche diodes136 and 136'. The back-to-back diodes 136 and 136' are particularly useful when there is a biphasic pulse waveform as shown in FIG. 2.

FIG. 9 is very similar to FIG. 8, except that the external antenna 120 has been omitted for clarity. Shown are the two diodes of FIG. 8 wired in series opposing configuration. This is also a very useful configuration for biophasic pulses as previously described in FIG. 2.

FIG. 10 is a schematic diagram very similar to FIG. 9 except that the back-to-back series connected diodes 136 and 136' are shown as a single chip 138.

FIG. 11 is very similar to FIGS. 9 and 10 except that the Zener diode 140 back-to-back symbol is used. The TVS device 134 in the present invention can be any type of diode, avalanche diode, transorb, or the like.

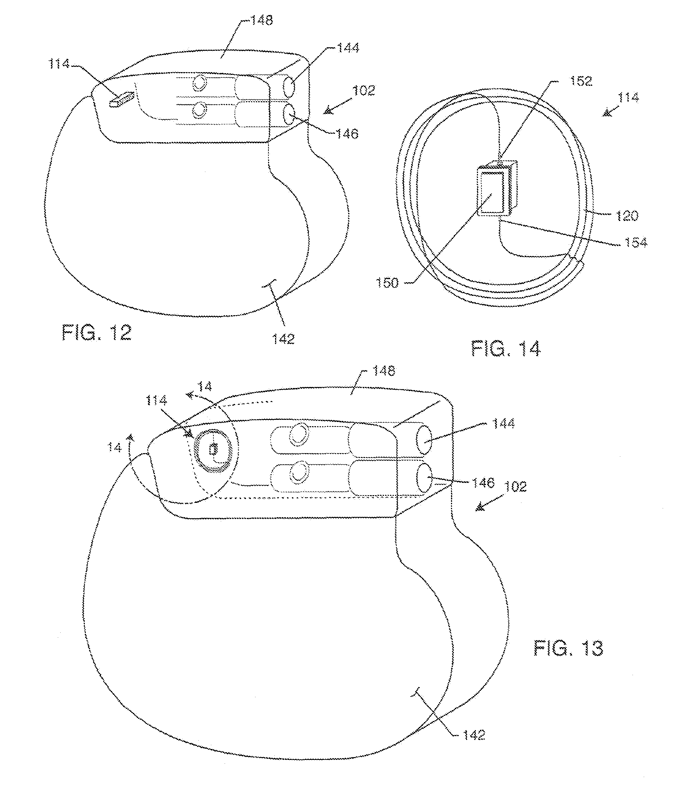

FIG. 12 is an isometric view of a typical AIMD 102, such as a cardiac pacemaker. Cardiac pacemakers typically have a metallic housing 142 which can be of titanium, stainless steel or the like. This metallic housing 142 is laser welded shut and generally contains a hermetic feedthrough terminal for passage of lead wires into the interior of the metallic housing 142. Hermetic feedthrough terminals are well known in the art and are generally laser welded into the metallic housing 142. The cardiac leads (not shown) are generally routed to connectors 144, 146. The connectors 144, 146 provide a convenient location to plug in the leads which are routed to the heart for pacing and biologic sensing. The connector assembly 144 and 146 is generally encapsulated within a molded non-metallic, i.e., plastic or ceramic, header block148, as shown. Usually, this header block 148 is of clear casting materials which are well known in the art. Opaque thermal setting or chemically setting materials may also be used. Such molded header blocks are common in the industry and are designated by ISO Standards IS-1, DF-1 or IS-4 or the equivalent. A non-hermetically sealed RFID tag 114 is encapsulated within the molded header block 148 of the AIMD.

The reason one would place the RFID tag 114 in the header block is that the header block materials are non-metallic and are therefore transparent to electromagnetic energy from an RFID reader. This is particularly advantageous if the RFID frequency were to be at 13.56 MHz or above. For low frequency RFID tags (LF) that operate typically at 125 to 135 kHz range, the RFID tag could be in the header block or even inside the titanium housing of an AIMD. Obviously, if the RFID chip and its associated antenna were in the hermetically sealed titanium housing 142, then the present invention embodying a biocompatible multi-turn loop antenna connected to a hermetically sealed RFID chip would not be required. However, to achieve optimum read range, it's preferable that the RFID tag 114 and its associated antenna 120 not be inside the electromagnetic shielded housing of an AIMD.

In accordance with the present invention, in FIG. 13 one can see that the RFID tag 114 has been embedded in header block 148 and is connected to a multiple-turn antenna 120. Read range is important in the present application. The read range should not be too excessive (for example, several meters) because of the possibility of creating electromagnetic interference (picking up stray tags and so on).

FIG. 14 shows a hermetically sealed package 150 containing the RFID chip 118 and the TVS circuit 134 protection diodes. There are biocompatible electrical connections 152 and 154 between the antenna 120 and the hermetic seal assembly terminals 150. These would typically be laser welds or brazes of all biocompatible materials or biocompatible solders or conductive polymers. In other words, no non-biocompatible solder joint or other such non-biocompatible connection would be exposed to body fluids. An alternative would be to use a biocompatible thermally conductive adhesive.

Referring once again to FIG. 13, one can see that the present invention satisfies the need for long term human implant. The header block 148 is not considered by biomedical scientists to provide a long term or reliable hermetic seal. Over time, through bulk permeability, body fluids and water will penetrate readily through that entire structure. This is why there is a hermetic seal to make sure that body fluids can never penetrate to the sensitive electronic circuits of an AIMD, as further explained by U.S. Patent Publication No. US-2006-0212096 A1, the contents of which are incorporated herein. The same principle applies in the present invention in that the sensitive microelectronic RFID chip and its associated electrical connections must also be protected over the long term from body fluid intrusion. There are two reasons why this is important. First of all, moisture intrusion to the level of the RFID chip 118 will cause its sensitive components to short out through formation of metal dendrites or the like. In addition, the electronic RFID chip 118 contains materials that are not biocompatible. They may even contain dangerous toxic materials to the human body, such as lead, cadmium and the like. Accordingly, hermetically sealing the RFID chip 118 is essential.

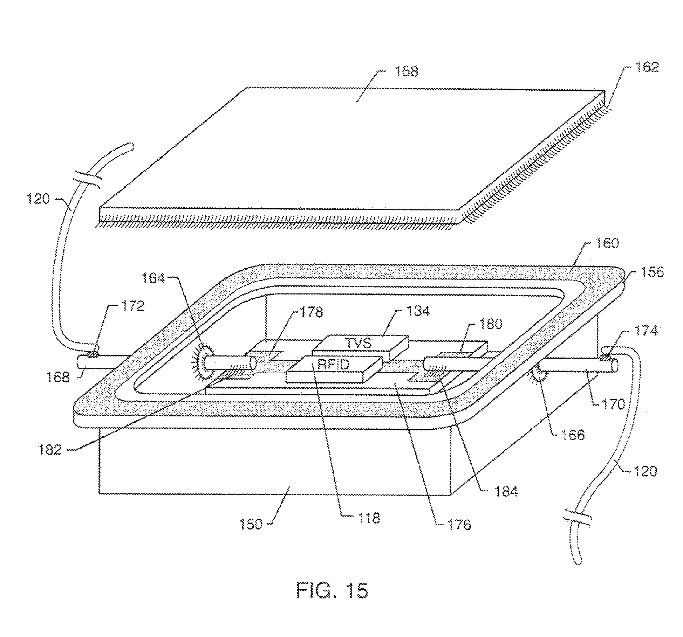

FIG. 15 shows an RFID chip 118 and the TVS diode(s) 134 inside the hermetically sealed housing 150. The housing 150 can be ceramic with a weld ring 156 and a ceramic lid 158 with a sputtered surface 160 as shown. These weld rings 156 would typically be titanium or platinum and they would be gold brazed 162 to the sputtered ceramic material 160. However, in a preferred embodiment, the entire housing 150 can simply be machined or made from powder metallurgy of titanium so that the entire structure is metal. Through this would penetrate hermetic seals 164 and 166 on each end. These hermetic seals, in a preferred embodiment, would be gold brazed ceramic seals. However, they could also be either fusion or glass compression seals. The terminal pins 168 and 170 extend out either end for convenient welding of the antenna 120 lead at locations 172 and 174 (the antenna itself is not shown). This is typically done by laser welding so that it would be entirely biocompatible. As previously mentioned, this could also be done with a biocompatible thermal-setting conductive adhesive. The RFID chip 118 may be attached to the inside of container 150 by means of a non-conductive substrate 176. Wire bond pads or metallizations 178 and 180 are formed on the substrate 176 and in conductive relation to the RFID chip 118 and the terminal pins 168 and 170, such as by gold braze or laser welds 182 and 184, as shown in FIG. 15. Since these electrical connections 182 and 184 will not be exposed to body fluids, they could also be comprised of solder or any other well-known non-biocompatible material. The electrical connections 172 and 174 can be eliminated. This would be accomplished by using a suitable biocompatible antenna wire, such as platinum or platinum-iridium, and routing it continuously through hermetic seals 164 and 166.

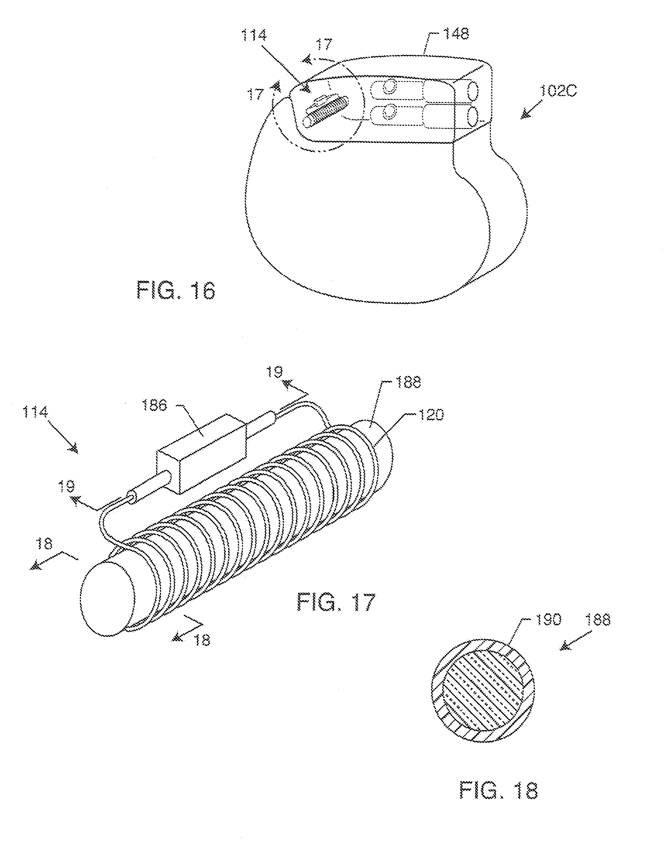

FIG. 16 is an isometric view of an AIMD such as a cardiac pacemaker 102 having a solenoid type RFID tag114, which is embedded within the header block 148 of the pacemaker. FIG. 17 is an enlarged view of the RFID tag 114. Shown is a hermetically sealed container 186 which houses the RFID chip 118 and its associated TVS protection diodes 136. The hermetically sealed container 186 is similar to that described in FIG. 15. There is also a multiple turn solenoid antenna 120 shown wrapped on a ferrite core 188. In a preferred embodiment, the ferrite core 188 would have a conformal insulative coating 190 as shown in FIG. 18. The hermetically sealed container 186 can be rectangular, cylindrical, or any shape.

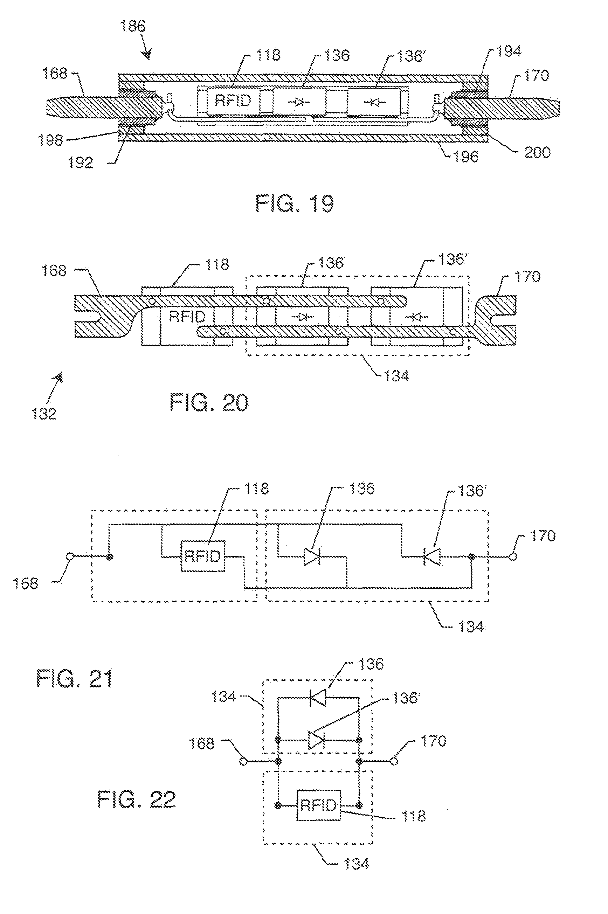

FIG. 19 is a sectional view of the hermetically sealed container 186 taken generally from section 19-19 of FIG. 17. Shown are either glass or gold brazed alumina hermetic seals 192 and194. In general, this is constructed by a "ship in the bottle" technique. That is, the RFID chip 118 and its associated TVS diodes 136 and 136' are all preassembled outside of the overall hermetically sealed housing 196. The hermetically sealed housing can be of ceramic, glass, or any biocompatible metals, such as platinum or titanium. When the electronic assembly, consisting of the RFID chip 118 and the TVS diodes 136 and 136' is inserted, then a laser weld 198 and 200 is performed on each end to hermetically seal the overall housing.

FIG. 20 illustrates the circuit connections of FIG. 19, and FIG. 21 is the electrical schematic diagram of FIG. 19. FIG. 21 shows the RFID microchip 118 which is now protected by a TVS circuit 134 consisting of parallel connected back-to-back diodes 136 and 136'. In this configuration, the avalanche voltage would only be limited by the forward bias voltage drop of the two diodes 136 and 136'. This would generally be from 0.65 to 0.7 volts. In a more practical application, these would be series connected Zener diodes so that their avalanche voltage could be set a higher voltage level.

FIG. 22 is the electrical schematic diagram of FIG. 21 that is redrawn in a more traditional format. It is obvious from FIG. 22 that the TVS circuit 134 is wired in parallel with the RFID chip 118.

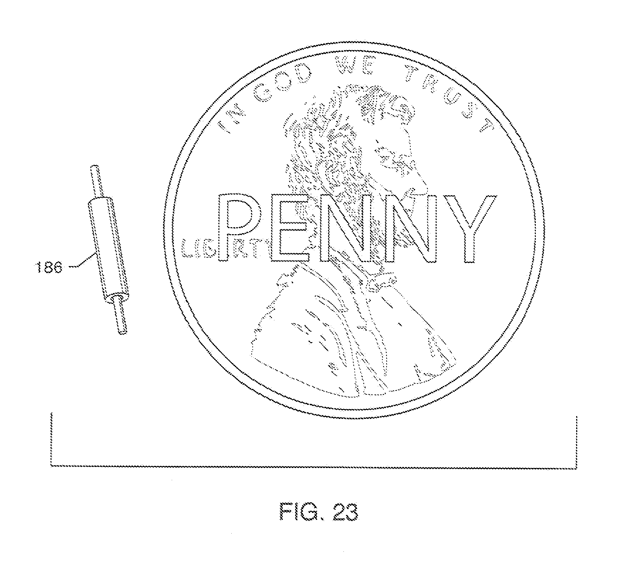

FIG. 23 shows how small the hermetically sealed container 186of FIG. 18 really is compared to a United States penny.

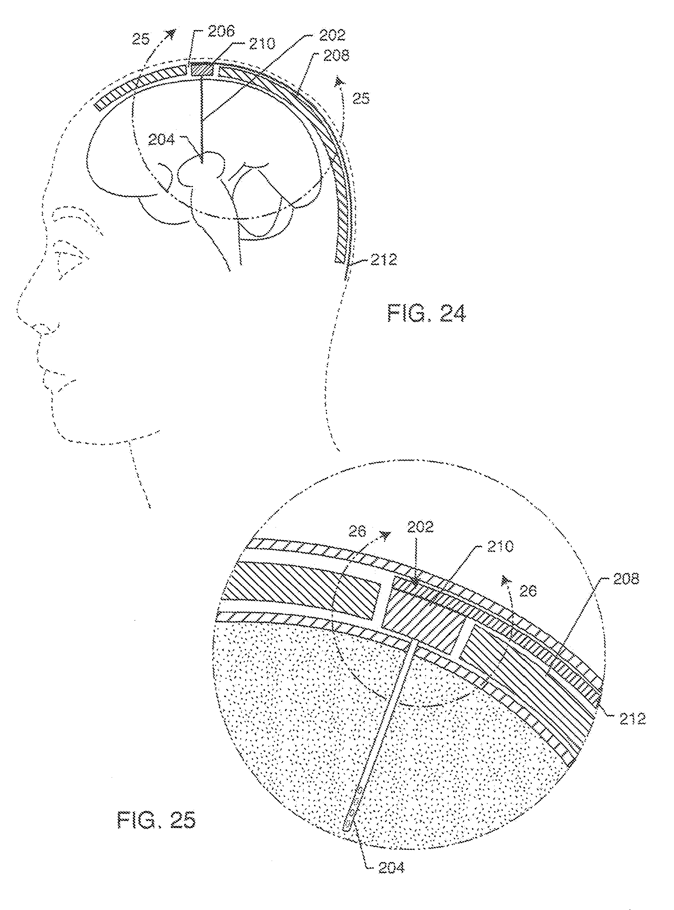

FIG. 24 is a cross-sectional view of a human head and skull showing an implanted deep brain stimulation electrode assembly202. The electrodes that are in contact with deep brain tissue are generally in the area designated by204. There is a burr hole 206 formed in the skull 208 which houses a hermetically sealed package 210. The hermetically sealed package 210 is affixed to the electrodes 204 and also to implanted leadwires 212. The implanted leadwires 212 are routed down the back of the patient's skull and neck by tunneling all the way to the pectoral area to an active implantable medical device (not shown). FIG. 25, taken generally from area 25-25 from FIG. 24, shows the deep brain electrode assembly 202 and also the hermetically sealed package 210.

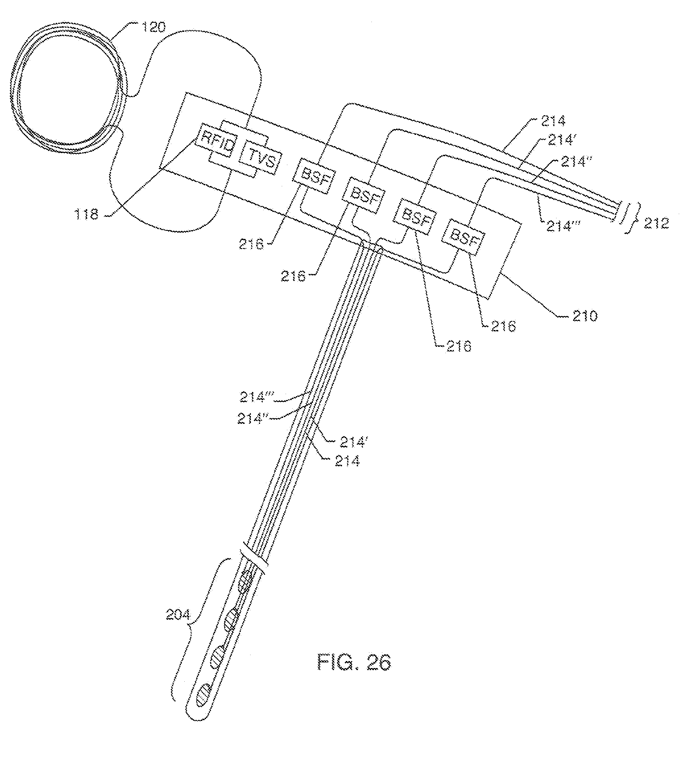

FIG. 26 is taken generally from area 26-26 from FIG. 25. An RFID chip 118 is shown routed to an external antenna 120 which would be typically located underneath the patient's skin, but above the skull. The RFID chip 118 could contain important information about the patient, or even the MRI compatibility of the deep brain electrodes 204. In accordance with the present invention, a transient voltage suppressor TVS 134 is shown wired in parallel with both the RFID microchip 118 and the RFID antenna120. Also shown are quadpolar circuits 214 each having as associated bandstop filter 216. The purpose of the bandstop filter is thoroughly described in U.S. Patent No. 7,363,090, the contents of which are incorporated herein.

From the foregoing, it will be appreciated that the present invention relates to a transient voltage suppression (TVS) circuit associated with an implanted RFID chip. The TVS circuit comprises, generally, an implanted RFID chip, an antenna associated with the RFID chip, and a transient voltage suppressor electrically connected in parallel to both the RFID chip and the antenna. A hermetically sealed biocompatible container is provided which is suitable for long-term exposure to body tissue or body fluids, in which the RFID chip and the transient voltage suppressor are disposed. The antenna preferably comprises a biocompatible material also suitable for long-term exposure to body tissue or body fluids. The transient voltage suppression circuit protects the sensitive RFID microchip from damage or shorting out in the presence of an over-voltage such as that caused by hospital, diagnostic or surgical equipment or by an automatic external defibrillator (AED).

Although several embodiments of the present invention have been described in detail for purposes of illustration, various modifications of each may be made without departing from the spirit and scope of the invention. Accordingly, the invention is not to be limited, except as by the appended claims.

## Claims

1. A transient voltage suppression (TVS) circuit for an implanted RFID tag, comprising:
an implantable RFID chip;
an antenna associated with the RFID chip; and
a transient voltage suppressor electrically connected in parallel to both the RFID chip and the antenna.

2. The TVS circuit of claim 1, wherein the transient voltage suppressor comprises an array of diodes.

3. The TVS circuit of claim 2, wherein the array of diodes comprises back-to-back diodes.

4. The TVS circuit of claim 1, wherein the transient voltage suppressor comprises at least one Zener diode.

5. The TVS circuit of claim 4, wherein the at least one Zener diode comprises back-to-back or series opposing Zener diodes.

6. The TVS circuit of claims 1, 2 or 4, wherein the antenna comprises a biocompatible material suitable for long-term exposure to body tissue or body fluids.

7. The TVS circuit of claim 6, including a hermetically sealed biocompatible container suitable for long-term exposure to body tissue or body fluids, in which the RFID chip and the transient voltage suppressor are disposed.

8. The TVS circuit of claim 7, wherein the hermetically sealed biocompatible container is disposed within a header for an active implantable medical device (AIMD).

9. The TVS circuit of claim 8, wherein the AIMD comprises a hearing device, a cochlear implant, a piezoelectric sound bridge transducer, a neurostimulator, a brain stimulator, a cardiac pacemaker, a left ventricular assist device, an artificial heart, a drug pump, a bone growth stimulator, a urinary incontinence device, a pain relief spinal cord stimulator, an anti-tremor stimulator, a cardioverter defibrillator, a congestive heart failure device, or a cardiac resynchronization therapy device.

10. The TVS circuit of claim 7, wherein the antenna is disposed about the hermetically sealed biocompatible container.

11. The TVS circuit of claim 7, wherein the RFID chip and the transient voltage suppressor are mechanically disposed inline within the hermetically sealed biocompatible container.

12. The TVS circuit of claim 1, wherein the RFID chip is associated with an implantable sensor or stimulator.

13. The TVS circuit of claim 12, wherein the implantable sensor or stimulator comprises a deep brain sensor or stimulator.
